(19) Europäisches Patentamt
European Patent Office
Office européen des brevets

(11) EP 0 697 021 B1

# (12) EUROPÄISCHE PATENTSCHRIFT

(45) Veröffentlichungstag und Bekanntmachung des Hinweises auf die Patenterteilung:
**05.07.2000 Patentblatt 2000/27**

(21) Anmeldenummer: **95909783.3**

(22) Anmeldetag: **25.02.1995**

(51) Int. Cl.[7]: **C07D 495/04**, C07K 14/435, C07K 14/36, G01N 33/53 // (C07D495/04, 333:00, 235:00)

(86) Internationale Anmeldenummer:
**PCT/EP95/00690**

(87) Internationale Veröffentlichungsnummer:
**WO 95/23800 (08.09.1995 Gazette 1995/38)**

## (54) PHOTOAKTIVIERBARE BIOTINDERIVATE UND DEREN EINSATZ ZUM ENTSTÖREN VON IMMUNOASSAYS

PHOTO-ACTIVATABLE BIOTIN DERIVATIVES AND THEIR USE IN SUPPRESSING INTERFERENCE IN IMMUNO ASSAYING

DERIVES PHOTOACTIVABLES DE LA BIOTINE ET LEUR UTILISATION POUR LA SUPPRESSION DES INTERACTIONS DANS LES DOSAGES IMMUNOLOGIQUES

(84) Benannte Vertragsstaaten:
**AT CH DE ES FR GB IE IT LI NL**

(30) Priorität: **05.03.1994 DE 4407423**

(43) Veröffentlichungstag der Anmeldung:
**21.02.1996 Patentblatt 1996/08**

(73) Patentinhaber:
**Roche Diagnostics GmbH**
**68298 Mannheim (DE)**

(72) Erfinder:
- **HUBER, Erasmus**
  **D-86923 Finning (DE)**
- **WIEDMANN, Michael**
  **D-82377 Penzberg (DE)**
- **DONIE, Frédéric**
  **D-82377 Penzberg (DE)**

(56) Entgegenhaltungen:
**EP-A- 0 155 854** **EP-A- 0 331 068**
**DE-A- 4 302 241**

- **JOURNAL OF PHOTOCHEMISTRY AND PHOTOBIOLOGY, A:CHEMISTRY, Bd.57, Nr.1-3, 1991, NL Seiten 331 - 342 U. HENRIKSEN ET AL 'Azidobenzoyl, azidoacridinyl, diazocyclopentadienylcarbonyl and 8-propyloxypsoralen photobiotinylation reagents. Syntheses and photoreactions with DNA and protein'**
- **Methods of Immunological Analysis (Hrsg.: R.F. Masseyeff, W.H. Albert, N.A. Staines), VCH, Weinheim, 1993, Bd. 3, Seiten 264 - 288**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 697 021 B1

## Beschreibung

Die Erfindung betrifft neue photoaktivierbare Biotinderivate, deren Herstellung, deren Verwendung zur Inaktivierung von Streptavidin oder Avidin, die Verwendung des inaktivierten Streptavidins zur Entstörung von Immunoassays und ein Verfahren zum Nachweis eines Analyten unter Verwendung des inaktivierten Streptavidins.

Immunologische Nachweismethoden haben in den letzten Jahren eine große Bedeutung erlangt. Mit ihnen kann die Gegenwart von Arzneimitteln, Hormonen, Proteinen, infektiösen Organismen und insbesondere spezifischen Antikörpern in biologischen Proben schnell und genau nachgewiesen werden. Bei allen immunologischen Nachweismethoden kommt es zu einer spezifischen Bindungsreaktion zwischen einem ersten spezifischen Bindungspartner, der Substanz, die nachgewiesen werden soll ("Analyt") und einem zweiten spezifischen Bindungspartner, der spezifisch mit dem Analyten reagiert oder bindet. Analyt und spezifischer Analytbindungspartner, die sogenannten Partner eines spezifischen Bindungspaares, bilden dabei ein spezifisches Bindungspaar, im allgemeinen einen Komplex zwischen einem Antigen und einem Antikörper oder Antikörperfragment. Dabei können mehr als ein Analyt oder ein Bindungspartner in jeder Reaktion miteinander reagieren. Diese spezifischen Bindereaktionen werden auf verschiedene Weise detektiert. Im allgemeinen ist ein Teilnehmer der spezifischen Bindereaktion markiert. Übliche Markierungsmethoden sind Radioisotope, Chromogene, Fluorogene. Enzymmarkierung oder Substanzen, die wiederum ein spezifisches Bindepaar bilden können (z.B. Biotin / Streptavidin). Bei heterogenen Immunoassays ist einer der Bindungspartner an eine Festphase immobilisiert.

Ein schwerwiegendes Problem bei Immunoassays ist, daß zwischen spezifischen Bindungspartnern des Immunoassays und der Probe, der in der Probe enthaltenden zusätzlichen Bestandteile und ggf. der Festphase unerwünschte Wechselwirkungen und unspezifische Bindereaktionen erfolgen können. Derartige Wechselwirkungen bewirken im allgemeinen eine Erhöhung des Hintergrundsignals und auch eine stärkere Streuung der Signale und damit eine verringerte Sensitivität und Spezifität des betreffenden Testes.

Sowohl durch unspezifische Wechselwirkungen mit dem markierten Bindungspartner als auch durch unspezifische Bindungen von Testkomponenten und Probenbestandteilen können falschpositive Messungen resultieren, d.h., es wird aufgrund des falsch erhöhten Meßsignals auf die Anwesenheit eines Analyten auch bei dessen Abwesenheit geschlossen.

Es wurden verschiedene Versuche unternommen, diese unspezifischen Wechselwirkungen in Immunoassays zu reduzieren. Seit langem ist bekannt, daß unterschiedliche Kohlenhydratkomponenten und unterschiedliche Proteine, Proteingemische oder Proteinfraktionen sowie deren Hydrolysate unspezifische Wechselwirkungen zwischen den Testkomponenten und dem Analyten im Immunoassay reduzieren können (beispielsweise Robertion et al., Journal of Immun. Meth. 26, 1985, 195; EP-A-260 903; US-A-4,931,385).

Der Einsatz von Proteinrohfraktionen und Rohhydrolysaten hat den Nachteil, daß durch die darin enthaltenen Bestandteile wiederum andere Störungen des Tests ausgelöst werden können. Enzymatisch produzierte Hydrolysate können zudem mit den bei der Herstellung verwendeten Proteasen kontaminiert sein und weisen in der Regel keine einheitliche Qualität auf, da sich die Spaltung nur schwer steuern läßt. Proteasekontaminationen können Testkomponenten angreifen und schon in geringen Mengen zur Beeinträchtigung der Testfunktion und Lagerstabilität führen.

Zur Verringerung unspezifischer Wechselwirkungen in Immunoassays wurde auch der Einsatz von chemisch modifizierten Proteinen, insbesondere von succinylierten oder acetylierten Proteinen beschrieben (US-A-5,051,356; EP-A-0 525 916). Mit diesen Substanzen können jedoch viele der falsch-positiven Ergebnisse bei Tests auf Antikörper aus Serum nicht vermieden werden.

EP-A-0 331 068 und WO 91/06 559 beschreiben den Einsatz von polymerisiertem Immunoglobulin, insbesondere IgG, zur Reduktion spezifischer Störfaktoren wie zum Beispiel Rheumafaktoren. Es lassen sich damit aber nicht alle störenden Wechselwirkungen zufriedenstellend ausschalten. Außerdem kann der Zusatz von unspezifischem humanem Immunoglobulin (monomere oder polymere Antikörper oder deren Fragmente) in Test auf humane Antikörper zu einer Erhöhung des Leerwertes führen. Ferner ist die Gewinnung von humanen oder tierischem IgG aufwendig und teuer.

Aufgabe der Erfindung war es daher, neue Entstorsubstanzen zur Verfügung zu stellen, die eine bessere Entstörung von unspezifischen Wechselwirkungen bei Immunoassays bewirken als aus dem Stand der Technik bekannt. Unter unspezifischen Wechselwirkungen sind alle Wechselwirkungen zwischen Komponenten des Verfahrens zu verstehen, die zu Verfälschungen des Messergebnisses führen können. Die Entstörsubstanzen sollen falsch-positive Analysenergebnisse vermeiden, besonders bei der Analyse von Antikörpern. Insbesondere soll eine Störung bei der Verwendung von Avidin oder Strepravidin als einem Bindepartner in einem Immunoassay vermieden werden.

Gelöst wurde diese Aufgabe durch spezifisch durch neue photoaktivierbare Biotinderivate inaktiviertes Avidin oder Streptavidin. Durch die Verwendung dieser Substanzen konnte überraschenderweise eine Entstorung bei Immunoassays, insbesondere bei Immunoassays, bei denen Avidin oder Streptavidin als ein Bindepartner eingesetzt wird, erzielt werden.

Gegenstand der Erfindung sind neue photoaktivierbare Biotinderivate der Formel I

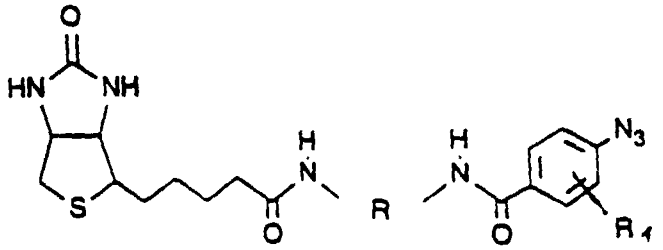

I

wobei R = $[(CH_2)_nO]_q$-$[(CH_2)_r$-$O]_t$-$(CH_2)_p$
mit n.r=2,3; q-t= 1-4 ; p =1-4,
wobei die Summe aller $CH_2$-Gruppen höchstens 10 ist
und wobei R1 als Ein- oder Mehrfachsubstituent Wasserstoff. $C_1$-$C_5$-Alkyl. $NH_2$, COOH, F, Cl oder Br bedeutet.

Besonders bevorzugt sind als R die folgenden Ketteneinschübe:

- $(CH_2)_3$-O-$(CH_2)_4$-O-$(CH_2)_3$-
-$(CH_2)_2$-O-$(CH_2)_2$-O-$(CH_2)_2$-

Ein weiterer Gegenstand der Erfindung ist die Verwendung der erfindungsgemaßen photoaktivierbaren Biotinderivate zur Inaktivierung von Streptavidin oder Avidin durch kovalente Kopplung, wodurch ein Komplex gemäß Formel II gebildet wird. Die Inaktivierung von Streptavidin wird so durchgefuhrt, daß die erfindungsgemaßen Biotinderivate zusammen mit Streptavidin oder Avidin kontaktiert werden. Durch die sehr hohe Bindungsaffinitat von Streptavidin zu Biotin wird Biotin an Streptavidin gebunden. Nach der Absattigung der Biotinbindestellen mit dem erfindungsgemaßen Biotinderivaten wird die Photoreaktion initiiert und so Biotin kovalent am aktiven Zentrum fixiert. Dabei kuppelt die photoaktivierbare Gruppe des Biotins mit einem Aminosaurerest des Streptavidins außerhalb des aktiven Zentrums und bindet so das Biotin fest und kovalent an die Rezeprorstelle des Streptavidins. Als Photoquelle kann beispielsweise eine Hg-Dampflampe dienen, die Bestrahlungswellenlänge liegt insbesondere zwischen 250 und 450 nm. Die Zeitdauer der Bestrahlung kann zwischen 1 min. und 10 h liegen, bevorzugt zwischen 5 und 30 min.

Unter Avidin oder Streptavidin sind naturlich vorkommendes gereinigtes Protein oder rekombinantes Avidin oder Streptavidin zu verstehen und deren chemisch modifizierte Derivate.

Es können alle Derivate der nativen Formen verwendet werden, die in der Lage sind, freies oder über die Carboxylgruppe kovalent konjugiertes Biotin zu binden. Solche Derivate können beispielsweise durch chemische Modifizierung wie Alkylierung mit Alkylhalogeniden, Acylierung mit Carbonsäurechloriden oder -estern, Oxidation von Zuckerresten (Seitenketten) mit z.B. Periodaten oder durch Deletion von einzelnen Aminosäuren oder Aminosäureblöcken innerhalb der Primärsequenz mittels enzymatischen oder gentechnischen Methoden hergestellt werden. Auch Oligomere oder Polymere von SA/Avidin (wobei auch SA mit Avidin gemischt sein kann), erzeugt durch chemische Vernetzung (z.B. mittels bifunktioneller Linker) sind möglich.

Gegenstand der Erfindung sind weiterhin erfindungsgemäß inaktiviertes Avidin oder Streptavidin der Formel II

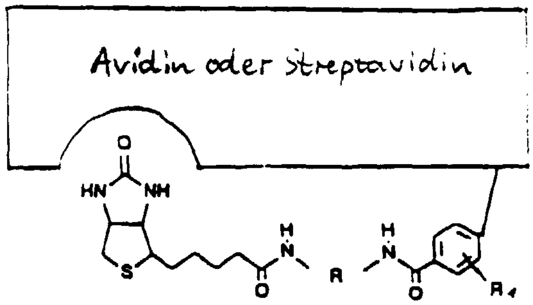


II

in der R und $R_1$ die vorne gegebene Bedeutung haben, wobei $R_1$ zusätzlich noch $NO_2$ bedeuten kann und bei dem Biotin an das aktive Zentrum von Streptavidin oder Avidin gebunden und zusätzlich über eine kovalente Bindung außerhalb des aktiven Zentrums an Streptavidin oder Avidin gebunden ist.

Photoaktivierbare Biotinderivate sind bekannt. In EP-A-0 155 854 und EP-A-0 187 332 werden azid-substituierte Phenyle / Nitrophenyle beschrieben, die mit einem aminhaltigen Linker an Biotin gekoppelt sind. Im Boehringer Mannheim Biochemica Katalog, Ident. Nr. 1292633 bzw. 1292641 wird ein photoreaktives Biotinderivat der Formel

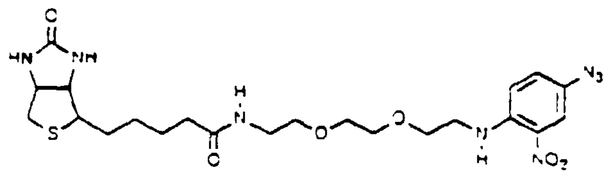

beschrieben.

Diese Biotinderivate werden jedoch nur zur Markierung von DNA / RNA Molekülen, von Proteinen allgemein und von Kohlenhydraten beschrieben. Eine Kupplung speziell an Streptavidin ist nicht beschrieben und wäre auch nicht für den in diesen Literaturstellen beschriebenen Zweck der Markierung sinnvoll, da Streptavidin selbst eine Bindungsstelle für Biotin enthält und somit keine freie Markierung mit Biotin möglich ist.

Ein weiterer Gegenstand der Erfindung ist die Verwendung des erfindungsgemäß inaktivierten Avidins oder Streptavidins zur Entstörung von Immunoassays. Besonders geeignet ist inaktiviertes Avidin oder Streptavidin zur Entstörung von Immunoassays, bei denen als eine Bindekomponente Avidin oder Streptavidin eingesetzt wird. Solche Immunassay sind beispielsweise aus U.S. Don et al., J. Histochem. Cytochem. 27 (1979), 1131 - 1139 und Bayer und Wilchek, Analytical Biochemistry 171 (1988), 1-32 bekannt. Die Störungen können beispielsweise durch Antikörper gegen Avidin oder Streptavidin, die teilweise im humanen Serum vorkommen, verursacht werden. Aber auch in Immunoassays, bei denen kein Avidin oder Streptavidin eingesetzt wird, bringt das erfindungsgemäße Entstörmittel eine vorteilhafte Wirkung. In diesen Immunoassays hat sich die Vewendung von inaktiviertem Streptavidin, das an ein weiteres Molekül gebunden ist, wie beispielsweise RSA oder Poly-RSA, als besonders vorteilhaft erwiesen.

Ein weiterer Gegenstand der Erfindung ist ein immunologisches Verfahren zur Bestimmung eines Analyten in einer Probe durch

1. Kontaktieren der Probe mit

    a) erfindungsgemäß inaktiviertem Avidin oder Strepavidin gemäß Formel II,
    b) einem oder mehreren spezifischen Bindepartnern des Analyten und

2. Messung des gebildeten Komplexes aus Analyt und spezifischen Bindepartner als ein Maß für die Anwesenheit des Analyten.

Als Analyt können alle Substanzen dienen, die mit mindestens einem spezifischen Bindepartner spezifisch zu einem Komplex reagieren, wie zum Beispiel Haptene, Antigene oder Antikörper. Bei dem Nachweis von Antikörpern, insbesondere Autoantikörpern, ist das erfindungsgemäße Verfahren besonders geeignet.

Als Probe dienen im allgemeinen Körperflüssigkeiten wie Blut, Plasma, Serum, Speichel oder Urin.

Als spezifischer Bindepartner kann jeder biologische oder chemische Bindepartner dienen, der spezifisch

den Analyten zu binden vermag und mit diesem einen Komplex ausbilden kann. Hierzu zählen Antikörper, Antikörperfragmente, Antigene, Haptene, Hormone, Avidin, Biotin oder Derivate davon. Bevorzugt werden in der vorliegenden Erfindung als Bindepartner des Analyten Antikörper oder Antigene oder deren Fragmente eingesetzt.

Zum Nachweis des Komplexes aus Analyt und spezifischem Bindepartner können alle dem Fachmann gängigen Methoden eingesetzt werden. Es können homogene Verfahren, bei dem alle Bindepartner im Verfahren in löslicher Form vorkommen, beispielsweise Präzipitationsverfahren mit tubidimetrischer oder nephelometrischer Bestimmung des gebildeten Komplexes oder Immunoassay nach dem CEDIA-, EMIT- oder FPIA-Prinzip verwendet werden. Geeignet sind ebenfalls heterogene Verfahren, bei denen mindestens ein Reagenz an eine feste Phase gebunden ist. Beispiele hierfür sind Agglutinationstest, bei denen ein Partner eines Bindepaares beispielsweise an Latex gebunden ist, Sandwichassays, ELISA zum Nachweis von spezifischen Antikörpern oder immunometrische Assays. Außer bei dem Präzipitationsverfahren ist bei allen diesen Verfahren mindestens einer der spezifischen Bindepartner markiert. Die Markierung kann direkt ein meßbares Signal liefern, beispielsweise ein Radioisotop, ein Chemilumineszenz, Fluoreszenz oder Elektrochemilumineszenzmarker oder ein gefärbter Partikel, beispielsweise ein Metallsolpartikel, oder gefärbter oder ungefärbter Latex. Die Markierung kann auch ein indirektes Signal liefern, beispielsweise eine Enzymmarkierung wie Peroxidase, Glucoseoxidase, β-Galaktosidase oder alkalische Phosphatase.

Die Immunoassays können ebenfalls mittels Teststreifen oder Biosensoren durchgeführt werden, insbesondere wenn einer der Bindepartner über Streptavidin an die Festphase gekoppelt ist. Auch Immunoassays nach dem Prinzip der Plasmonenresonanz können erfindungsgemäß entstört werden.

Oft wird ein Reagenz des Verfahrens zum Nachweis des Analyten über ein spezifisch bindendes Bindepaar, beispielsweise Avidin bzw. Streptavidin / Biotin an die Festphase gekoppelt. Der Vorteil hierbei ist, daß die Festphase universell bei mehreren Nachweisverfahren eingesetzt werden kann. Auch ist es möglich, die Markierung über ein spezifisches Bindepaar an eine Komponente des Assays zu binden. Beispielsweise kann ein Enzym an Avidin oder Streptavidin gekoppelt sein und der Bindepartner, beispielsweise ein Antikörper, kann biotinyliert sein. Beispiele für solche Nachweisverfahren sind dem Fachmann bekannt. Für diese Fälle ist das erfindungsgemäß inaktivierte Avidin oder Streptavidin besonders geeignet.

Zur Durchführung des Verfahrens wird der Analyt mit den einzelnen Testkomponenten und inaktiviertem Avidin oder Streptavidin inkubiert und der Immunoassay durchgeführt. Die Konzentration des erfindungsgemäßen Entstörmittels liegt dabei bevorzugt zwischen 0,0001 und 1% (m/v), bevorzugt zwischen 0,01 und 1% (m/v).

Die einzelnen Reaktionskomponenten des Verfahrens zum Nachweis eines Analyten werden zweckmäßigerweise in Form einer Testkombination oder eines Testkits angeboten.

Ein weiterer Gegenstand der Erfindung ist daher ein Testkit zum Nachweis eines Analyten in einer Probe mit

a) einem erfindungsgemäß inaktivierten Avidin oder Streptavidin gemäß Formel II und
b) mindestens einem spezifischen Bindepartner des Analyten.

Weiterhin können alle weiteren zur Durchführung des Nachweisverfahrens notwendigen Reagenzien wie Puffer, Detergentien, Markierung,, Hilfsstoffe zum Nachweis der Markierung, wie Enzymsubstrate, Festphase usw. enthalten sein. Bevorzugt wird das erfindungsgemäße Entstörmittel und der oder die Bindepartner des Analyten in getrennten Behältnissen abgepackt. Das Entstörmittel kann aber auch zu dem oder den Bindepartnern des Analyten direkt zugesetzt werden.

Ein weiterer Gegenstand der Erfindung ist ein Verfahren zur Herstellung der photoaktivierbaren Biotinderivate der Formel I. Das Verfahren wird so durchgeführt, daß Biotin mit an sich bekannten Kondensationstechniken über seine Carboxylfunktion an eine primäre oder sekundäre Aminogruppe einer Diaminoverbindung gekuppelt wird. Danach wird die noch freie Aminofunktion über eine weitere Kondensationsreaktion oder Substitutionsreaktion (z.B. aromatische nucleophile Substituenten) an die photoaktivierbare Gruppe gebunden. Dieses Syntheseschema ist wahlweise umkehrbar.

<u>Beispiel 1:</u>

<u>Herstellung von photoaktivierbarem Biotin</u>

**Synthese von Biotin-[8-(4-azidobenzoyl)amino-3,6-dioxaoctyl]amid (Biotin-DADOO-AB)(4)**

1.50 g (4 mmol) Biotinoyl-1,8-diamino-3,6-dioxaoctan (Biotin-DADOO, Boehringer Mannheim Nr. 1112074) werden unter Rühren in 50 ml frisch destilliertem DMF gelöst. Zu der Lösung gibt man nacheinander 1.04 g (4 mmol) N-Hydroxysuccinimidyl-(4-azidobenzoat) (HSAB, Boehringer Mannheim Nr. 1140051) und 0.55 ml (4 mmol) Triethylamin und läßt 2 h bei 20°C rühren. Anschließend wird das Losungsmittel am Rotationsverdampfer unter Ölpumpenvakuum entfernt und das verbleibende Rohprodukt durch Chromatographie an Kieselgel aufgereinigt. Hierzu wird in

möglichst wenig Chloroform/Methanol 2/1 (v/v) unter leichtem Erwärmen auf ca. 40°C gelöst und auf eine Kieseigel 60 (Fa.Merck) Säule (4 x 60 cm) aufgetragen. Man eluiert mit Chloroform/Methanol 2/1 (v/v) und sammelt in 50 ml Fraktionen. Die das reine Produkt enthaltenden Fraktionen werden durch DC (System wie unten beschrieben) ermittelt und vereinigt. Das Lösungsmittel wird am Rotationsverdampfer entfernt und der halbfeste Rückstand mit ca. 50 ml Diisopropylether digeriert. Das feinkristalline, farblose Produkt wird abgesaugt und über Nacht im Vakuumtrockenschrank (0.1-0.15 bar/40°C) getrocknet.

| | |
|---|---|
| Ausbeute; | 1.24 g (60% d.Th.) |
| DC: | Kieselgel 60(Merck) $F_{254}$, Chloroform/Methanol 2/1 (v/v); $R_f$= 0.71. |
| $^1$H-NMR(100MHz/$d_6$-DMSO): | δ(ppm) = 1.20-1.65(m, 6H); 2.07(tr, 2H); 2.60-3.65 (m, 15H); 4.05-4.20 (m; 2H); 6.38(d.br, 2H), 7.20 (d. 2H), 7.62 (tr.br, 2H); 7.91(d, 2H); 8.53 (tr,br, 2H). |
| UV($CH_3OH$): | λ(max) = 267nm |
| IR(KBr): | υ = 2125 $cm^{-1}$ |

Figur 1

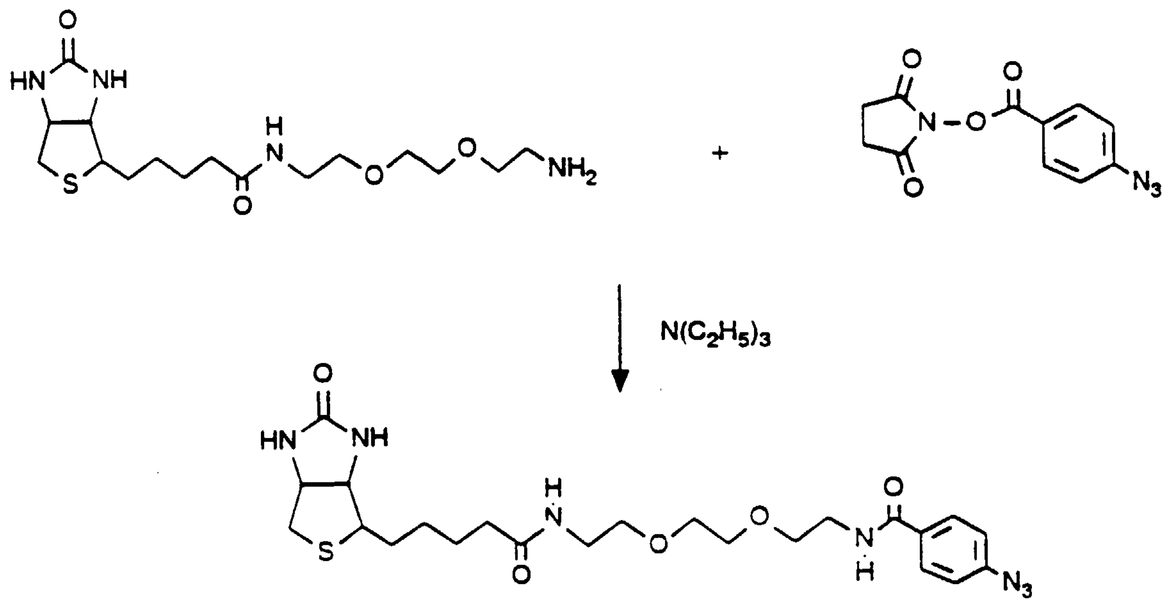


Beispiel 2:

Herstellung von inaktiviertem Streptavidin

**Prinzip:**

Streptavidin wird mit einem photoaktivierbaren Biotinderivat (z.B. Biotin-DADOO-AB) umgesetzt und zur Abtrennung des freien, nicht gebundenen Biotins dialysiert. Durch Bestrahlen mit Hg-Dampf-Lampe (350 - 700 nm)

wird die Photoreaktion initiiert und das Biotin kovalent im Bindezentrum von Streptavidin fixiert.

**Detaillierung:**

Zu 1 g Streptavidin wird bei einer Proteinkonzentration von 20 mg / ml in PBS-Puffer pH 7,5 ein 10-facher molarer Überschuß von Biotin-DADOO-AB-Reagenz zugesetzt (3,5 ml einer 25 mg/ml Biotin-DADOO-AB-Stammlösung in DMSO). Nach Zugabe wird 2 Stunden bei 25° C unter Lichtausschluß gerührt.

Freies, nicht gebundenes Biotinderivat wird durch Dialyse (20 Std. 4° C) gegen >500-faches Volumen PBS-Puffer, pH 7,5 unter Lichtausschluß vollständig (nicht mehr nachweisbar) abgetrennt. Der Ansatz wird dann bei einer Schichtdicke der Lösung von < 5 cm mit einer Hg-Dampf-Lampe (350 - 700 nm) unter Rühren für 20 min. bestrahlt und anschließend erneut gegen > 500-faches Volumen PBS-Puffer, pH 7,5 dialysien (16-18 Stunden, 4° C).

Beispiel 3:

Reinigung von inaktiviertem Streptavidin

**Prinzip:**

Durch Chromatographie an Rinderserumalbumin-Biotin- und/oder Streptavidin-Adsorber auf Spherosil-Basis wird inaktiviertes SA von SA mit verbliebener Restaktivität (Biotinbindung), von verbliebenem freiem Biotin oder von SA mit kovalentem an der Oberfläche zugänglichem Biotin (im Gegensatz zum in der Bindetasche fixiertem Biotin) gereinigt.

**Detaillierung:**

In den Reaktionsansatz wird pro 10 mg Protein 1 ml Streptavidin-Spherosil-Adsorber, äquilibiert in PBS-Puffer, pH7,5, gegeben und 2 Std. bei RT gerührt. Die Herstellung des Adsorbers erfolgt nach üblichen Verfahren durch Kopplung von Streptavidin an Gluthardialdehydaktiviertes Amino-Spherosil.

Die Suspension wird dann in eine Säule überführt und das Säulenmaterial mit PBS-Puffer pH 7,5 gewaschen. Dabei wird am Auslauf der Säule über UV-Monitor bei $E_{280nm}$ der Proteingehalt verfolgt. Es wird bis zur Proteinfreiheit gewaschen. Der proteinhaltige Durchlauf wird in einer Fraktion aufgefangen.

In den proteinhaltigen Durchlauf des Streptavidin-Adsorbers wird dann pro 10 mg Protein 1 ml Rinderserumalbumin-Biotin (RSA-Bi)-Spherosil-Adsorber, äquilibriert in PBS-Puffer, pH 7,5, gegeben und 2 Std. bei RT gerührt. Die Herstellung des Adsorbers erfolgt nach üblichem Verfahren durch Kopplung von RSA-Bi an Gluthardialdehyd-aktiviertes Amino Spherosil.

Die Suspension wird in eine Säule überführt und mit PBS-Puffer, pH 7,5 gewaschen. Dabei wird am Auslauf der Säule über UV-Monitor bei $E_{280nm}$ der Proteingehalt verfolgt. Der proteinhaltige Durchlauf enthält das Produkt und wird in einer Fraktion aufgefangen. Das Produkt (inaktiviertes SA) wird auf eine Proteinkonzentration von 20 mg/ml konzentriert und nach Abfüllung lyophilisiert.

Referenzbeispiel 4

Synthese von Biotin-[2-(4-Azidobenzoyl)aminoethyl]amid (Biotin-EDA-AB) 4a

**N-(4-Azidobenzoyl)ethylendiamin 2a**

2.6 g (10 mmol) HSAB **1** werden in 70 ml THF gelöst und innerhalb 1 h unter Rühren und Eiskühlung zu einer Lösung von 6.01 g (100 mmol) Ethylendiamin in THF getropft. Danach entfernt man das Eisbad und läßt die Temperatur langsam auf Raumtemperatur kommen. Das Lösungsmittel wird am Rotationsverdampfer abgezogen und das überschüssige Ethylendiamin größtenteils am Hochvakuum entfernt. Das verbleibende Rohprodukt wird durch präparative Säulenchromatographie an Kieselgel 60 (Fa.Merck; Säule 4 x 68 cm, Laufmittel: Essigester/Eisessig/Wasser 6/3/1 (v/v/v)) gereinigt. Die entsprechenden Fraktionen werden gesammelt und eingedampft. Das Produkt **2a** wird in ca. 100 ml Methanol aufgenommen und nach Abziehen des Lösungsmittels am Rotationsverdampfer als leicht gelblicher Feststoff erhalten.

Ausbeute: 2.92 g (enthält ca. 2-3 Äquivalente $CH_3COOH$).
DC: Kieselgel 60 $F_{254}$ (Merck); Essigester/Eisessig/Wasser 6/3/1 (v/v/v); $R_f$= 0.49.

**Biotin-[2-(4-Azidobenzoyl)aminoethyl]amid 4a**

1.03 g (5 mmol) **2a** werden in 40 ml dest. DMIF suspendiert und unter Rühren bei 20°C mit 1.4 ml Triethylamin versetzt. Anschließend gibt man 1.71g (5 mmol) Biotin-N-hydroxysuccinimidester (Boehringer Mannheim Nr.734250) **3** zu und läßt weitere 3 h bei 20°C rühren. Danach wird das Lösungsmittel am Rotationsverdamfer (Ölpumpenvakuum, Wasserbad 50°C) entfernt und der Eindampfrückstand über Nacht mit gesättigter $NaHCO_3$- Lösung digeriert. Das Produkt **4a** wird abgesaugt, mit Wasser gewaschen und im Exsikkator getrocknet.

Ausbeute: 1.02 g (47% d.Th.) weißlich gelbes Pulver.

DC: Kieselgel 60 $F_{254}$ (Merck); Essigester/Methanol 3/1 (v/v) + 1% Eisessig; $R_f$= 0.31

$^1$H-NMR(100MHz/$d_6$-DMSO): δ(ppm)= 1.20-1.65(m, 6H); 2.08(tr, 2H); 2.60-3.45 (m, 7H), 4.05-4.20 (m; 2H); 6.40(d,br, 2H), 7.20 (d, 2H); 7 89(d, 2H); 7.90 tr,br, 1H); 8.49 (tr,br, 1H).

UV($CH_3OH$): λ(max) = 268 nm

IR(KBr): υ = 2117 $cm^{-1}$

Referenzbeispiel 5

Synthese von Biotin-[2-(4-Azidobenzoyl)aminohexyl]amid (Biotin-HMDA-AB) 4b

**N-(4-Azidobenzoyl)hexamethylendiamin 2b**

2.6 g (10 mmol) HSAB **1** werden in 70 ml THF gelöst und innerhalb 1 h unter Rühren und Eiskühlung zu einer Lösung von 11.62 g (100 mmol) Hexamethylendiamin in THF getropft. Danach entfernt man das Eisbad und läßt die Temperatur langsam auf Raumtemperatur kommen. Das Lösungsmittel wird am Rotationsverdampfer abgezogen und das überschüssige Hexamethylendiamin größtenteils am Hochvakuum entfernt. Das verbleibende Rohprodukt wird 20 min mit 100 ml Wasser digeriert und anschließend über Nacht im Exsikkator getrocknet. **2b** wird als leicht gelblich-brauner Feststoff erhalten.

Ausbeute: 2.09 g (80 % d.Th).
DC: Kieselgel 60 $F_{254}$ (Merck); Essigester/Eisessig/Wasser 6/3/1 (v/v/v); $R_f$= 0.50.

**Biotin-[2-(4-Azidobenzoyl)aminohexyl]amid 4b**

1.30 g (5 mmol) **2b** werden in 40 ml dest. DMF suspendiert und unter Rühren bei 20°C mit 1.4 ml Triethylamin versetzt. Anschließend gibt man 1.71g (5 mmol) Biotin-N-hydroxysuccinimidester **3** zu und läßt weitere 3 h bei 20°C rühren. Danach wird das Lösungsmittel am Rotationsverdamfer (Ölpumpenvakuum, Wasserbad 50°C) entfernt und der Eindampfrückstand über Nacht mit gesättigter $NaHCO_3$ -Lösung digeriert. Das Produkt **4b** wird abgesaugt, mit Wasser gewaschen und im Exsikkator getrocknet.

Ausbeute: 1.71 g (70 % d.Th.) weißlich gelbes Pulver.

DC: Kieselgel 60 $F_{254}$ (Merck); Essigester/Methanol 3/1 (v/v) + 3 % Eisessig; $R_f$= 0.38.

$^1$H-NMR(100MHz/$d_6$-DMSO) δ(ppm) = 1.20-1.75(m, 14H); 2.06(tr, 2H); 2.60-.40 (m, 7H); 4.05-4.20 (m; 2H); 6.39(d,br, 2H); 7.19 (d, 2H); 7.73 (tr,br, 1H); 7.90(d, 2H); 8.44 (tr,br, 1H).

UV($CH_3OH$): λ(max) = 267 nm

IR(KBr): υ = 2121 $cm^{-1}$

Referenzbeispiel 6

Synthese von N-[3-(4-Azidobenzoyl)aminopropyl]-N-(3'-biotinoylaminopropyl)-methylamin Acetat 4c (Stand der Technik gemäß EP-A-0 155 854)

**N-(3-Aminopropyl)-N-[3'-(4-azidobenzoyl)aminopropyl]-methylamin 2c**

0.52 g (2 mmol) HSAB **1** werden in 20 ml THF gelost und innerhalb 45 min unter Rühren und Eiskühlung zu einer Lösung von 0.52 g (2 mmol) N,N-Bis-(3-aminopropyl)methylamin in 15 ml THF getropft. Danach entfernt man das Eisbad und läßt die Temperatur langsam auf Raumtemperatur kommen. Das Lösungsmittel wird am Rotationsverdampfer abgezogen und das überschüssige N,N-Bis-(3-aminopropyl)methylamin unter Erwärmen des Kolbens größtenteils am Hochvakuum entfernt. Das verbleibende Rohprodukt wird 20 min mit 100 ml Ether digeriert und anschließend über Säulenchromatographie an Kieselgel 60 (4 x 68 cm; Eluent: Chloroform/Methanol/Ammoniak 2/2/1 (v/v/v)) gereinigt. Die entsprechenden Fraktionen werden gesammelt, das Lösungsmittel entfernt und das verbleibende Produkt **2c** mehrere Stunden am Hochvakuum getrocknet.

Ausbeute: 380 mg (66 % d.Th) leicht gelblich-braunes Öl.

DC: Kieselgel 60 $F_{254}$ (Merck); Chloroform/Methanol/Ammoniak 2/2/1 (v/v/v)); $R_f$= 0.82

**N-[3-(4-Azidobenzoyl)aminopropyl]-N-(3'-biotinoylaminopropyl)-methylamin Acetat 4c**

290 mg (1 mmol) **2c** werden in 25 ml frisch destilliertem DMF gelöst und mit 0.17 ml Triethylamin und 342 mg (1 mmol) Biotin-N-hydroxysuccinimidester **3** versetzt. Die Lösung wird 1 h bei 20°C gerührt und anschließend am Hochvakuum eingedampft. Das Rohprodukt wird über Säulenchromatographie an Kieselgel 60 (4 x 45 cm; Eluent: Essigester/Eisessig/Wasser 5/5/2 (v/v/v)) gereinigt. Die entsprechenden Fraktionen werden vereinigt und das Lösungsmittelgemisch am Rotationsverdampfer entfernt. Der ölige Rückstand wird in Methanol gelöst, eingedampft und mit Diisopropylether digeriert. Abschließend wird das Produkt **4c** mindestens 6 h am Hochvakuum getrocknet.

Ausbeute: 405 mg (70 % d.Th.) zähes, leicht gelblich-braunes Öl.
DC: Kieselgel 60 $F_{254}$ (Merck); Essigester/Eisessig/Wasser 5/5/2 (v/v/v); $R_f$= 0.38
$^1$H-NMR(100MHz/$d_6$-DMSO): $\delta$(ppm)= 1.20-1.85 (m, 10H); 1.89 (s, 3H); 2.05(tr, 2H); 2.20-3.40 (m, 14H); 4.05-4.20 (m; 2H); 6.39(d,br, 2H); 7.19 (d, 2H); 7.80 (tr,br. 1H); 7.90 (d,2H); 8.52 (tr,br, 1H).
UV($CH_3OH$): $\lambda$(max) = 269 nm
IR($CHCl_3$): $\upsilon$ = 2112 $cm^{-1}$

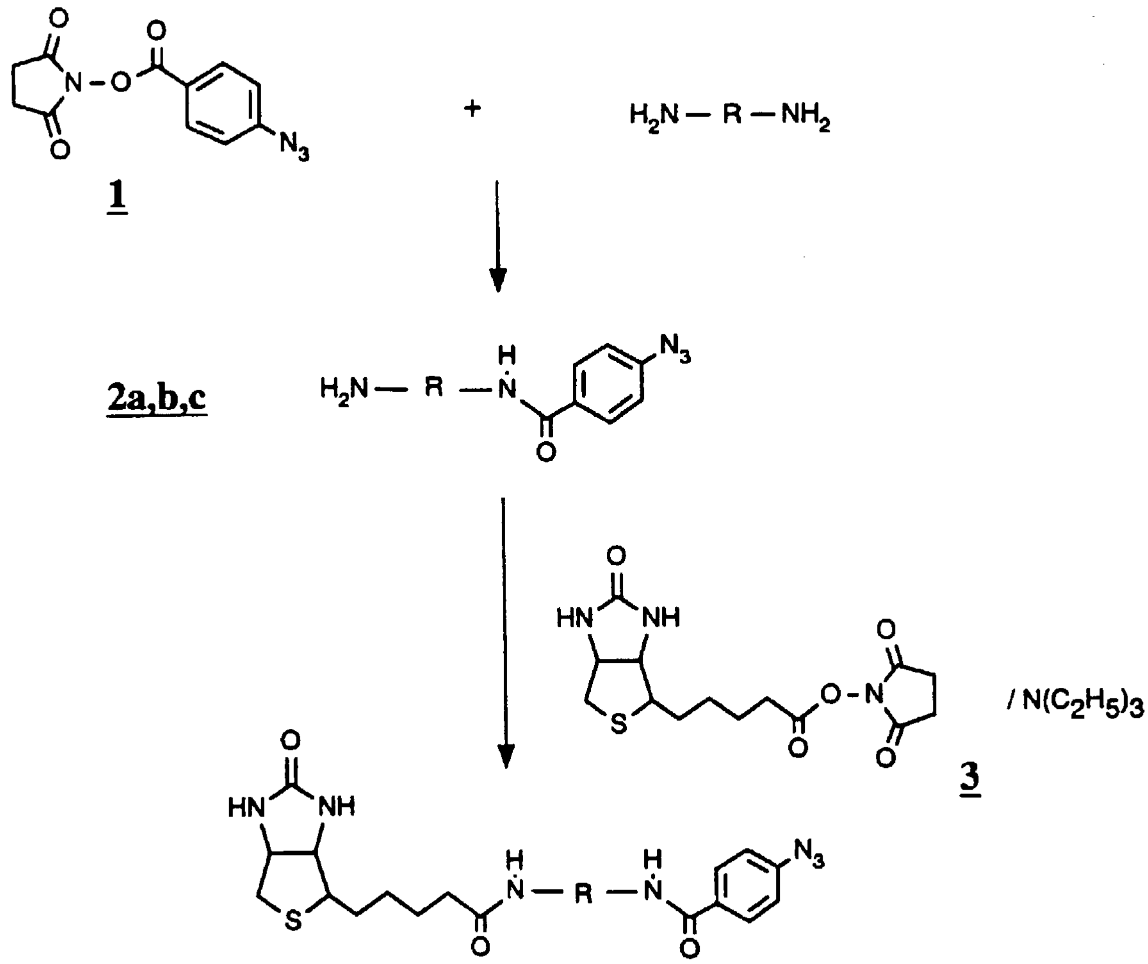


1

2a,b,c

3

4a,b,c

| | |
|---|---|
| a: | R = $(CH_2)_2$ |
| b: | R = $(CH_2)_6$ |
| c: | R = $(CH_2)_3N(CH_3)(CH_2)_3$ |

Beispiel 7

Entstörung eines HCV-Testes mit Biotin(photoaktiviert)-SA.

**Testprinzip:**

2-Schritt-Sandwichassay mit Streptavidinfestphase (Testführung und Reagenzien wie bei Boehringer Mannheim, Enzymuntest Anti-HIV)

| | |
|---|---|
| 1. Schritt: | Biotinylierte Peptide plus Probe |
| 2. Schritt: | Reaktion der wandgebundenen Antikörper mit einem Anti-human-IgG-POD Konjugat |
| 3. Schritt: | Indikatorreaktion mit ABTS als Substrat |

**Inkubationspuffer:**

Na-Phosphat 40 mmol/l, pH 7,4
NaCl 7,1 g/l
Konservierungsmittel
Plasma-Diagnostic-Based 200 g/l
HCV-Peptide aus der Core, NS4 und NS5 Region
± inaktiviertes Streptavidin nach Beispiel 1 und 2

**Konjugatpuffer:**

Na-Phosphat 40 mmol/l, pH 7,4
NaCl 7,1 g/l
Konservierungsmittel
Rinderalbumin 1 g/l
Rinder-IgG 4 g/l
Triton X 100 1 g/l
Anti-human IgG - POD 15 U/l

**Inkubationszeiten:**

| | |
|---|---|
| 1. Schritt: | 1 Stunde (Probe + Inkubationspuffer) |
| 2. Schritt: | 1 Stunde (+ Konjugatpuffer) |
| 3. Schritt: | 1 Stunde (Substratreaktion mit ABTS) |

**Proben:**

3 negative Serumproben (Referenz 1)
6 falsch positive Anti-HCV-Negativproben
3 positive Anti-HCV-Proben (Referenz 2)

**Volumina:**

Probe 20 µl
alle anderen Reagenzien jeweils 500 µl

**Testdurchführung:**

am ES 600 bei 25°C

**Substratmessung:**

Messung der Substratlösung bei 422 nm am ES 600 (Boehringer Mannheim GmbH)
Die Extinktionen sind in Tabelle 1 dargestellt.

Tabelle 1

| Proben | ohne inakt. SA im Inkubationspuffer | 20 µg/ml inakt. SA im Inkubationspuffer | Signalabnahme nach Zugabe von inakt. SA |
|---|---|---|---|
| Negativserum 1 | 0,036 | 0,027 | * |
| Negativserum 2 | 0,042 | 0,035 | * |
| Negativserum 3 | 0,078 | 0,076 | * |
| HCV-Negativserum 1 | 0,528 | 0,125 | 76% |
| HCV-Negativserum 2 | 0,467 | 0,106 | 77% |
| HCV-Negativserum 3 | 0,979 | 0,161 | 84% |
| HCV-Negativserum 4 | 0,499 | 0,094 | 81% |
| HCV-Negativserum 5 | 2,049 | 0,471 | 77% |
| HCV-Negativserum 6 | 0,427 | 0,065 | 85% |
| HCV Positivserum 1 | 1,747 | 1,645 | 6% |
| HCV Positivserum 2 | 1,161 | 1,076 | 7% |
| HCV Positivserum 3 | 1,104 | 1,026 | 7% |

* keine Angabe, da aufgrund des geringen Meßsignales nicht sinnvoll.

Beispiel 8

Vergleich von mit verschiedenen photoaktivierbaren Biotinen desaktiviertem Streptavidin.

Streptavidin wird mit photoaktivierbaren Biotinen gemäß Beispiel 2, 5 und 6 desaktivist und gemäß Beispiel 7 miteinander verglichen. Die mit Bi-HMDA-(HS) AB und Bi-D ADOO (HS) AB hergestellten Streptavidinreagenzien zeigen gegenüber dem mit einem Biotinderivat gemäß dem Stand der Technik hergestellten Streptavidin eine deutlich verbesserte Entstörwirkung (HS) steht für die Abgangsgruppe Hydroxysuccinimid

| Proben | ohne inakt. SA im Inkubtions- Puffer | Signalabnahme nach Zugabe von 0,05 mg / ml SA inaktiv in Inkubations-Puffer | | |
|---|---|---|---|---|
| | | Bi-HMDA-(HS)AB (4b) | Bi-DADOO-(HS)AB (4) | Bi-DAPMA-(HS)AB (4c) |
| Neg. Serum 1 | 1,237 | 95% | 96% | 80% |
| Neg. Serum 2 | 0,163 | 46% | 50% | 10% |
| Pos. Serum 1 | 3,945 | 10% | 9% | 12% |

**Patentansprüche**

1. Photoaktivierbares Biotinderivat der Formel I

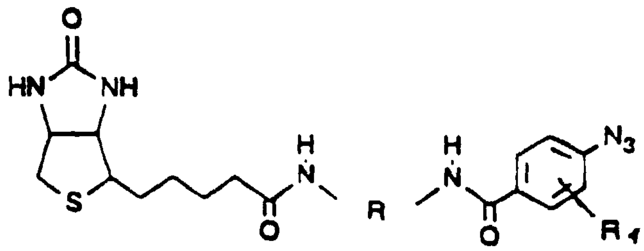

I

wobei $R = [(CH_2)_n\text{-}O]_q\text{-}[(CH_2)_r\text{-}O]_t\text{-}(CH_2)_p$
mit n=2,3; q+t = 1-4 ; p= 1-4; r= 2,3
wobei die Summe aller $CH_2$-Gruppen höchstens 10 ist
und wobei R1 als Ein- oder Mehrfachsubstituent Wasserstoff, $C_1$-$C_5$-Alkyl, $NH_2$, COOH, F, Cl oder Br bedeutet.

2. Photoaktivierbares Biotinderivat der Formel I, wobei

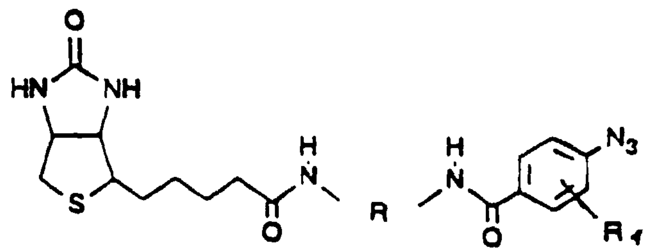

I

$R = [(CH_2)_n\text{-}O]_q\text{-} [(CH_2)_r \text{-} O]_t \text{-}(CH_2)_p$
mit n = 3, r = 4, q, t = 1 und p = 3.

3. Biotinderivat gemäß Anspruch 1, dadurch gekennzeichnet, daß n,r 2, q, t = 1, p = 2 ist.

4. Inaktiviertes Avidin oder Streptavidin der Formel II

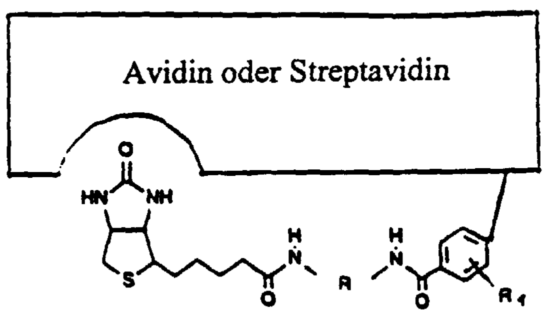


II

in der R und R1 die Bedeutung gemäß Anspruch 1 -3 haben, wobei R1 zusätzlich noch $NO_2$ bedeuten kann und

bei dem Biotin an das aktive Zentrum von Streptavidin oder Avidin und zusätzlich über eine kovalente Bindung außerhalb des aktiven Zentrums gebunden ist.

**5.** Verwendung des photoaktivierbaren Biotinderivates gemäß einem der Ansprüche 1 - 3 zur Inaktivierung von Streptavidin oder Avidin durch kovalente Kopplung, wodurch ein Komplex gemäß Anspruch 4 gebildet wird.

**6.** Verwendung von inaktiviertem Streptavidin gemäß Anspruch 4 zur Entstörung von Immunoassays.

**7.** Verwendung gemäß Anspruch 6, dadurch gekennzeichnet, daß zusätzlich auch Streptavidin als Bindepartner in dem Immunoassay verwendet wird.

**8.** Entstörmittel für Immunoassays, dadurch gekennzeichnet, daß es inaktiviertes Streptavidin gemäß Anspruch 4 enthält.

**9.** Verfahren zur immunologischen Bestimmung eines Analyten in einer Probe mit den Schritten

a) Kontaktieren der Probe mit inaktiviertem Streptavidin oder Avidin gemäß Anspruch 4 oder einem Entstörmittel gemäß Anspruch 8
b) gleichzeitig zuvor oder danach mit einem oder mehreren spezifischen Bindepartner des Analyten und
c) Messung des gebildeten Komplexes aus Analyt und spezifischen Bindepartnern als Maß für die Anwesenheit des Analyten.

**10.** Verfahren gemäß Anspruch 9, dadurch gekennzeichnet, daß mindestens eine Bindekomponente des Immunoassays über Avidin / Biotin oder Streptavidin / Biotin gekoppelt wird.

**11.** Testkit zur Bestimmung eines Analyten in einer Probe bestehend aus

a) inaktiviertem Streptavidin oder Avidin gemäß Anspruch 4 oder einem Entstörmittel gemäß Anspruch 8
b) mindestens einem spezifischen Bindepartner des Analyten.

**12.** Verfahren zur Herstellung eines photoaktivierbaren Biotinderivates gemäß einem der Ansprüche 1 - 3, dadurch gekennzeichnet, daß an eine Diaminoverbindung der Formel

$$NH_2\text{-}R\text{-}NH_2$$

die Carboxylgruppe von Biotin an einem Ende und eine photoaktivierbare Gruppe der Formel

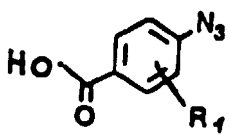

am anderen Ende kondensiert wird, wobei R und R1 die in den Ansprüchen 1 - 3 gegebene Bedeutung haben.

**Claims**

**1.** Photoactivatable biotin derivative of formula I

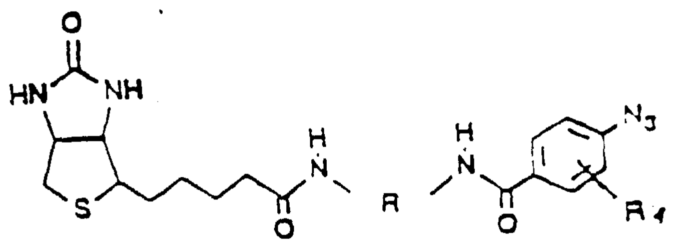

I

in which R = $[(CH_2)n\text{-}O]_q\text{-}[(CH_2)_r\text{-}O]_t\text{-}(CH_2)_p$
where n = 2,3; q+t = 1-4 ; p= 1-4; r = 2,3
wherein the sum of all $CH_2$ groups does not exceed 10
and wherein R1 as a single or multiple substituent denotes hydrogen, $C_1$-$C_5$ alkyl, $NH_2$, COOH, F, Cl or Br.

**2.** Photoactivatable biotin derivative of formula I

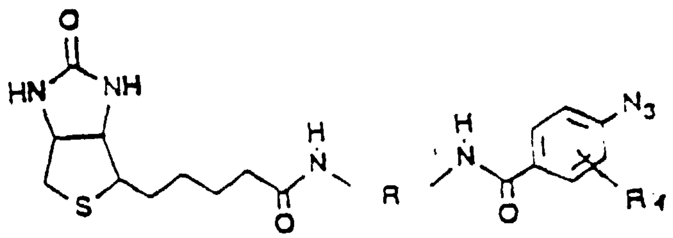

I

in which R = $[(CH_2)_n\text{-}O]_q\text{-}[(CH_2)_r\text{-}O]_t\text{-}(CH_2)_p$
where n = 3, r = 4, q,t = 1 and p = 3.

**3.** Biotin derivative as claimed in claim 1, wherein n,r = 2, q,t = 1, p = 2.

**4.** Inactivated avidin or streptavidin of formula II

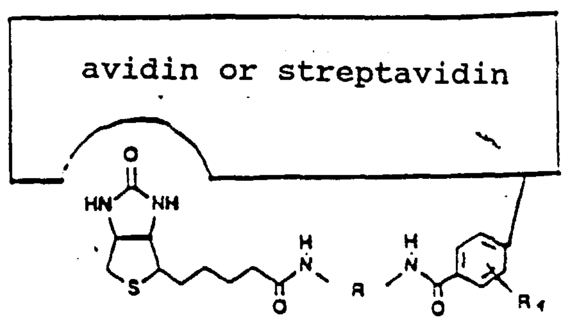


II

in which R and R1 have the meaning as claimed in claims 1 - 3, wherein R1 can also denote $NO_2$ and biotin is bound to the active centre of streptavidin or avidin and in addition is bound via a covalent bond outside the active centre.

**5.** Use of the photoactivatable biotin derivative as claimed in one of the claims 1 - 3 to inactivate streptavidin or avidin by covalent coupling resulting in formation of a complex as claimed in claim 4.

**6.** Use of inactivated streptavidin as claimed in claim 4 to reduce interference in immunoassays.

**7.** Use as claimed in claim 6, wherein streptavidin is also additionally used as a binding partner in the immunoassay.

**8.** Interference-reducing agent for immunoassays, wherein it contains inactivated streptavidin as claimed in claim 4.

**9.** Method for the immunological determination of an analyte in a sample comprising the following steps:

a) contacting the sample with inactivated streptavidin or avidin as claimed in claim 4 or with an interference-reducing agent as claimed in claim 8
b) simultaneously before or after with one or several specific binding partners of the analyte and
c) measuring the formed complex of analyte and specific binding partners as a measure for the presence of the analyte.

**10.** Method as claimed in claim 9, wherein at least one binding component of the immunoassay is coupled via avidin/biotin or streptavidin/biotin.

**11.** Test kit for the determination of an analyte in a sample comprising<

a) inactivated streptavidin or avidin as claimed in claim 4 or an interference-reducing agent as claimed in claim 8,
b) at least one specific binding partner of the analyte.

**12.** Process for the production of a photoactivatable biotin derivative as claimed in one of the claims 1 - 3, wherein the carboxyl group of biotin is condensed to one end and a photoactivatable group having the formula

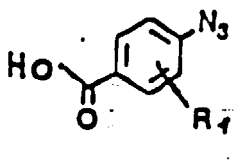

in which R and R1 have the meaning given in claims 1 - 3 is condensed to the other end of a diamino compound having the formula $NH_2$-R-$NH_2$.

**Revendications**

**1.** Dérivé photoactivable de la biotine, répondant à la formule I

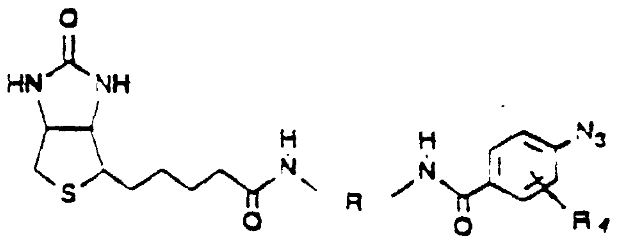

I

dans laquelle

R représente un groupe $[(CH_2)_n-O]_q-[(CH_2)_r-O]_t-(CH_2)_p$
où n = 2,3; q+t = 1-4 ; p = 1-4; r = 2,3,
la somme de tous les groupes $CH_2$ étant au maximum égale à 10,
et $R_1$ représentant, à titre de substituant unique ou multiple, un atome d'hydrogène, un groupe alkyle en $C_1$-$C_5$, un groupe $NH_2$, un groupe COOH, un atome de fluor, un atome de chlore ou un atome de brome.

2. Dérivé photoactivable de la biotine, répondant à la formule I

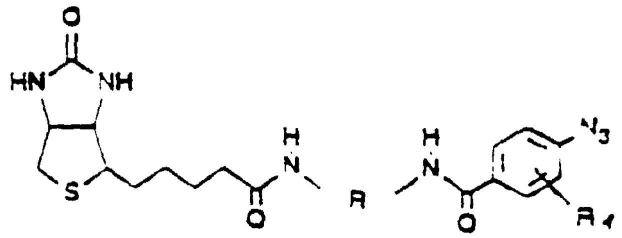

I

dans laquelle

R représente un groupe $[(CH_2)_n-O]_q-(CH_2)_r-O]_t-(CH_2)_p$
où n=3, r=4, q,t= 1 et p=3.

3. Dérivé de la biotine selon la revendication 1, caractérisé en ce que n,r = 2, q,t = 1, p=2.

4. Avidine ou streptavidine inactivée répondant à la formule II

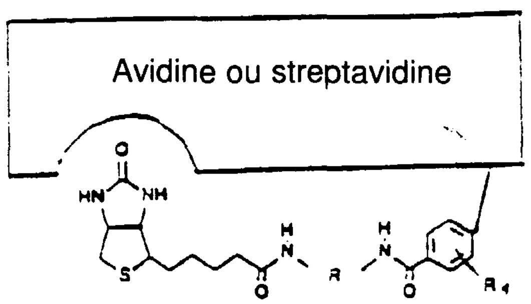


II

dans laquelle R et $R_1$ ont la signification selon les revendications 1 à 3, $R_1$ pouvant en outre représenter un groupe $NO_2$ et

dans laquelle de la biotine est liée au centre actif streptavidine ou avidine et en outre via une liaison covalente à l'extérieur du centre actif.

5. Utilisation du dérivé de la biotine photoactivable selon l'une quelconque des revendications 1 à 3 pour l'inactivation de la streptavidine ou de l'avidine par couplage covalent si bien que l'on obtient un complexe selon la revendication 4.

6. Utilisation de streptavidine inactivée selon la revendication 4 à des fins d'antiparasitage d'immunodosages.

7. Utilisation selon la revendication 6, caractérisée en ce qu'on utilise en outre de la streptavidine à titre de partenaire de liaison dans l'immunodosage.

8. Agent d'antiparasitage pour des immunodosages, caractérisé en ce qu'il contient de la streptavidine inactivée selon la revendication 4.

9. Procédé pour la détermination immunologique d'un analyte dans un échantillon comprenant les étapes consistant à:

a) mettre l'échantillon en contact avec de la streptavidine ou de l'avidine inactivée selon la revendication 4 ou avec un agent d'antiparasitage selon la revendication 8,

b) de manière simultanée, avant ou après cette mise en contact, le mettre en contact avec un ou plusieurs partenaires de liaison spécifiques de l'analyte, et

c) mesurer le complexe formé par l'analyte et par des partenaires de liaison spécifiques, comme mesure pour la présence de l'analyte.

**10.** Procédé selon la revendication 9, caractérisé en ce qu'au moins un composant de liaison de l'immunodosage est couplé via de l'avidine/biotine ou via de la streptavidine/biotine.

**11.** Nécessaire d'essai pour la détermination d'un analyte dans un échantillon, constitué par

a) de la streptavidine ou de l'avidine inactivée selon la revendication 4 ou un agent antiparasitage selon la revendication 8,

b) au moins un partenaire de liaison spécifique de l'analyte.

**12.** Procédé pour la préparation d'un dérivé de la biotine photoactivable selon l'une quelconque des revendications 1 à 3, caractérisé on ce que, sur un composé diamino répondant à la formule

$$NH_2\text{-}R\text{-}NH_2$$

le groupe carboxyle de la biotine est condensé à une extrémité et un groupe photoactivable répondant à la formule

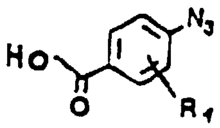

est condensé à l'autre extrémité, R et $R_1$ ayant la signification indiquée dans les revendications 1 à 3.